(19) Europäisches Patentamt
European Patent Office
Office européen des brevets

(11) **EP 2 185 062 B1**

(12) **EUROPÄISCHE PATENTSCHRIFT**

(45) Veröffentlichungstag und Bekanntmachung des Hinweises auf die Patenterteilung:
**19.03.2014   Patentblatt 2014/12**

(21) Anmeldenummer: 08785090.5

(22) Anmeldetag: **25.07.2008**

(51) Int Cl.:
*A61B 5/145* (2006.01)     *A61B 5/027* (2006.01)
*A61B 5/1459* (2006.01)     *A61B 5/00* (2006.01)
*A61M 25/00* (2006.01)     *A61M 25/01* (2006.01)

(86) Internationale Anmeldenummer:
**PCT/EP2008/006137**

(87) Internationale Veröffentlichungsnummer:
**WO 2009/015835 (05.02.2009 Gazette 2009/06)**

(54) **KATHETERSYSTEM MIT OPTISCHER SONDE UND VERFAHREN ZUR APPLIKATION EINER OPTISCHEN SONDE IN EIN KATHETERSYSTEM**

CATHETER SYSTEM HAVING AN OPTICAL PROBE AND METHOD FOR THE APPLICATION OF AN OPTICAL PROBE IN A CATHETER SYSTEM

ENSEMBLE CATHÉTER À SONDE OPTIQUE ET PROCÉDÉ POUR APPLIQUER UNE SONDE OPTIQUE DANS UN ENSEMBLE CATHÉTER

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **31.07.2007   DE 102007035847**

(43) Veröffentlichungstag der Anmeldung:
**19.05.2010   Patentblatt 2010/20**

(73) Patentinhaber: **UP-MED GmbH**
**81673 München (DE)**

(72) Erfinder:
• **PFEIFFER, Ulrich**
**81667 München (DE)**
• **MOULAS, Daniel**
**32547 Bad Oeynhausen (DE)**
• **KNOLL, Reinhold**
**81543 München (DE)**

(74) Vertreter: **Graf von Stosch, Andreas et al**
**Graf von Stosch**
**Patentanwaltsgesellschaft mbH**
**Prinzregentenstrasse 22**
**80538 München (DE)**

(56) Entgegenhaltungen:
**EP-A- 0 891 807     EP-A- 1 402 917**
**WO-A-85/02101     WO-A-97/05820**
**US-A- 4 718 423     US-A- 5 634 720**

**Beschreibung**

Die Erfindung betrifft ein Kathetersystem mit optischer Sonde und ein Verfahren zur Applikation einer optischen Sonde in ein Kathetersystem. Insbesondere betrifft die Erfindung einen Katheter mit Faseroptik und ein Verfahren zur passgenauen Einbringung der Faseroptik in den Katheter.

In der Intensivmedizin kommen häufig zentralvenöse Katheter (ZVK) mit mehreren Lumina, sogenannte Multilumen-ZVK, zur Anwendung. Mit diesen Multilumen-ZVK werden verschiedene Parameter ermittelt, Infusionslösungen, Blut und Blutderivate sowie Pharmaka über verschiedene Lumina zugeführt und Blutproben für blutgasanalytische hämatologische und biochemische Untersuchungen entnommen. Außerdem ist es bekannt, faseroptische Sonden über ein Faseroptiklumen einzubringen, um beispielsweise die zentralvenöse Sauerstoffsättigung (Scv02) zu messen.

In EP 1 402 917 ist ein Katheter mit optischen Fasern beschrieben. Das Kathetersystem ist zur gleichzeitigen, kontinuierlichen, gegenseitig unbeeinflussten Messung der zentralvenösen Sauerstoffsättigung geeignet. Es weist einen zentralvenösen Katheter mit einem Faseroptiklumen und eine in das Faseroptiklumen einführbare faseroptische Sonde zur Vornahme reflexionsoximetrischer Messungen auf. Zur Vermeidung einer Längsverschiebung der faseroptischen Sonde relativ zu dem Faseroptiklumen sind ein fest mit der faseroptischen Sonde verbundenes Anschlussstück und ein fest mit dem Katheter verbundenes Gegenstück vorgesehen, welche aneinander anschließbar sind.

Die internationale Patentanmeldung WO 2007/101508 A1 offenbart eine Messvorrichtung zur Messung der Sauerstoffsättigung, die zum Einsatz mit einem Zentralvenenkatheter vorgesehen ist, wobei die Vorrichtung ein Faseroptikbündel mit einem distalen Ende und mit einem proximalen Ende umfasst, wobei das Faseroptikbündel über afferente und efferente Lichtleitfasermittel zum Aussenden von Signalen und zum Empfangen von Signalen zum Erzeugen von venösen Sauerstoffsättigungsmessungen verfügt. Ferner weist die Messvorrichtung um das Faseroptikbündel angeordnete Hüllmittel zum Kapseln und zum Schützen des Faseroptikbündels auf, wobei das distale Ende des Faseroptikbündels exponiert bleibt. Die Messvorrichtung verfügt ferner über Verriegelungsmittel zum Festlegen des Faseroptikbündels in Bezug auf einen Katheter, in denen das Faseroptikbündel eingefügt ist, um die relative Anordnung zwischen dem Faseroptikbündel und dem Katheter bei Anordnung in situ während eines Sauerstoffsättigungsmessungsvorganges zu fixieren. Die Verriegelungsmittel weisen Hülsenmittel auf, die funktional an dem Verriegelungsmittel angebracht sind, um die Hüllmittel, die um das Faseroptikbündel herum angeordnet sind, zu schützen und steril zu halten.

Durch die fest vorkonfektionierte Länge der faseroptischen Sonde und der festen Verbindung der faseroptischen Sonde mit dem Anschlussstück ist eine genaue Abstimmung der einzelnen faseroptischen Sonde auf den zu verwendenden zentralvenösen Katheter erforderlich. Um eine Messung der zentralvenösen Sauerstoffsättigung über die Sonde zu realisieren, ragt die Spitze der Sonde über das distale Ende des ZVK zirka 25 mm ± 5 mm hinaus. Dadurch ist sichergestellt, dass die Sonde vom zentralvenösen Blut umströmt wird und eine Messung an diesem Punkt die zentralvenöse Sauerstoffsättigung ergibt. Gleichzeitig darf die Spitze der Sonde nicht im rechten Vorhof liegen, da sie dort zu Irritationen des Herzens führen würde.

Aufgrund der unterschiedlichen Länge einzelner ZVKs durch verschiedene Hersteller ist es daher erforderlich, dass für die verschiedensten Anwendungen unerschiedlich abgelängte faseroptische Sonden bereitgestellt werden, die für den jeweiligen ZVK von der Länge her so abgestimmt sind, dass diese Kriterien erfüllt werden. Wenn versehentlich eine zu kurze faseroptische Sonde eingesetzt wird, so kann der korrekte Wert der zentralvenösen Sauerstoffsättigung nicht ermittelt werden, da sich die Spitze der Sonde dann immer noch im ZVK befindet. Wenn eine zu lange faseroptische Sonde gewählt wird, besteht die Gefahr, dass die Sonde im rechten Vorhof zu liegen kommt, und zu Reizungen des Herzens bzw. dem Tod des Patienten führt. Eine zu weit aus dem ZVK herausragende Sonde wird eventuell nicht ausreichend geführt und kann dann leicht ungünstig positioniert werden z.B. an der Gefäßwand anliegen oder Knicken.

Aufgabe der vorliegenden Erfindung war es daher ein Kathetersystem mit insbesondere einer faseroptischen Sonde bereitzustellen, bei dem die Nachteile des Standes der Technik vermieden werden.

Die Aufgabe wird gelöst durch ein Kathetersystem aufweisend einen flexiblen, länglichen, zentralvenös applizierbaren Grundkörper (2), eine faseroptische Sonde (8), ein Faseroptiklumen zur Aufnahme der faseroptischen Sonde (8) und Befestigungsmittel (25) zur Vermeidung einer Längsverschiebung der faseroptischen Sonde (8) relativ zu dem Faseroptiklumen, wobei die Befestigungsmittel (25) lösbar sind, um eine Längsverschiebung der faseroptischen Sonde (8) relativ zu dem Faseroptiklumen zur Entnahme der optischen Sonde (8) zuzulassen, wobei die Befestigungsmittel (25) ein fest mit der faseroptischen Sonde (8) verbundenes Anschlussstück (7) und ein fest mit dem zentralvenös applizierbaren Grundkörper (2) verbundenes Gegenstück (6) aufweisen, welche aneinander anschließbar sind und wobei in angeschlossenem Zustand der Abstand zwischen distalem Ende (3) des zentralvenös applizierbaren Grundkörpers (2) und distalem Ende (14) der faseroptischen Sonde (8) durch die festen Verbindungen zwischen der faseroptischen Sonde (8) und dem Anschlussstück (7) sowie Grundkörper (2) und Gegenstück (6) auf einen vorbestimmten Wert eingestellt ist und wobei zusätzlich ein längenverstellbares Schaftstück (30) am Anschlussstück (7) vorgesehen ist, über das die Länge des Anschlussstückes (7) und damit die Länge des Faseroptiklumens variiert werden kann.

Ein Kathetersystem ist ein System bestehend aus verschiedenen Baugruppen zur Messung von Parametern eines Patienten, Applikation verschiedener Stoffe, Entnahme von Proben oder Einführung von Messsonden. Besonders bevorzugt sind zentralvenöse Katheter mit mehreren Lumina, sogenannte Multilumen-ZVK. Diese Katheter werden in das zentralvenöse Systeme eingebracht und verbleiben dort. Über Adapter kann der Operateur im nicht-sterilen Bereich über bevorzugt mehrere Anschlüsse Sonden einbringen, Blut entnehmen, Pharmaka verabreichen, etc.

Das Kathetersystem weist insbesondere einen Grundkörper auf, der bevorzugt flexibel ausgebildet ist. Dieser Grundkörper ist zentralvenös applizierbar, d. h., kann in das zentralvenöse System eingeführt werden und verbleibt dort. Der Grundkörper ist dann bevorzugt mit einem Adapter verbunden, der über mindestens ein Anschlussstück, bevorzugt drei Anschlussstücke verfügt, über die dann Sonden oder andere Systeme angeschlossen werden können.

Das Kathetersystem weist mindestens eine faseroptische Sonde auf, über die beispielsweise die zentralvenöse Sauerstoffsättigung über optische Messverfahren, insbesondere mittels Faseroptikreflexionsoximetrie kontinuierlich gemessen werden kann. Besonders bevorzugt weist die Faseroptiksonde einen Überzug, insbesondere einen dünnen flexibel ausgebildeten äußeren Schaft bestehend aus bio- und hämokompatiblem Material, wie z. B. Polyurethan, auf. Hierin ist dann die Faseroptik untergebracht, die vorzugsweise aus flexiblen Kunststofffasern besteht.

In dem Kathetersystem ist mindestens ein Faseroptiklumen vorgesehen, d. h. ein durchgehender Bereich, in dem die faseroptische Sonde eingeführt werden kann und bis zum distalen Ende des Grundkörpers durchgeschoben werden kann. Dieses Faseroptiklumen erstreckt sich somit über die Anschlussstücke, einen Adapter bis hin durch den Grundkörper. Besonders bevorzugt sind neben dem Faseroptiklumen auch noch weitere Lumina vorgesehen, besonders bevorzugt insgesamt 4 Lumina, insbesondere 5 Lumina.

Die Befestigungsmittel sind vorgesehen, um die in das Kathetersystem eingebrachte faseroptische Sonde fest mit dem Kathetersystem, insbesondere dem Adapter des Kathetersystems zu verbinden. Auf diese Weise wird eine Längsverschiebung der faseroptischen Sonde relativ zu dem Faseroptiklumen vermieden. Ein solches Befestigungsmittel kann bevorzugt eine Luer-Lock-Verbindung sein. Dadurch sind die Befestigungsmittel geeignet, die faseroptische Sonde in der gewünschten Position zu fixieren und diese Befestigungsmittel können dann wieder gelöst werden, um die faseroptische Sonde aus dem Kathetersystem zu entnehmen.

Die Befestigungsmittel weisen besonders bevorzugt ein fest mit der faseroptischen Sonde verbundenes Anschlussstück auf. Dieses Anschlussstück ist mit der Sonde fest verbunden, so dass ein Verschieben der Sonde relativ zu dem Anschlussstück nicht möglich ist. Als Gegenstück zu diesem Anschlussstück weisen die Befestigungsmittel bevorzugt ein fest mit dem zentralvenös applizierbaren Grundkörper verbundenen Gegenstück auf. Dieses Gegenstück kann beispielsweise an einem Adapter zwischen dem Grundkörper und dem eigentlichen Gegenstück angebracht sein. Durch dieses Gegenstück hindurch zieht sich besonders bevorzugt das Faseroptiklumen bis hin zum distalen Ende des flexiblen Grundkörpers. Das Anschlussstück und das Gegenstück sind aneinander anschließbar, besonders bevorzugt durch ein Luer-Lock und hierdurch wird der Abstand zwischen dem distalen Ende des zentralvenös applizierbaren Grundkörpers einerseits und dem distalen Ende der faseroptischen Sonde im eingebrachten Zustand andererseits fixiert.

Gemäß der vorliegenden Erfindung ist bevorzugt ein längenverstellbares Schaftstück vorgesehen, das besonders bevorzugt am Anschlussstück ausgebildet ist. Über das längenverstellbare Schaftstück ist nun die Länge des Anschlussstückes variierbar bzw. einstellbar. Durch das längenverstellbare Schaftstück kann damit die Länge des Faseroptiklumens variiert werden. Da die faseroptische Sonde fest mit dem Anschlussstück verbunden ist, führt das längenverstellbare Schaftstück dazu, dass die Sonde innerhalb des Faseroptiklumens um die Länge, um die das längenverstellbare Schaftstück variierbar ist, verschoben werden kann. Auf diese Weise ist es über das längenverstellbare Schaftstück möglich, verschieden lange faseroptische Sonden für einen applizierten ZVK zu verwenden, indem, bevorzugt vor der Applikation der Sonde, über das längenverstellbare Schaftstück am Anschlussstück die überragende Länge der faseroptischen Sonde in Bezug auf das vorgegebene ZVK optimal einstellbar ist. Damit können für verschiedenste handelsübliche ZVKs eine Standard-faseroptische Sonde eingesetzt werden, deren Länge dann über das längenverstellbare Schaftstück am Anschlussstück für den entsprechenden ZVK passend eingestellt wird.

Bevorzugt kann zusätzlich während des Einführens der Faseroptik mit dem angeschlossenen Auswertegerät für die Messung der Sauerstoffsättigung die Intensität der reflektierten optischen Strahlung gemessen und die Plausibilität des erhaltenen Sauerstoffsättigungswertes überprüft werden. Bei Eintritt der Sondenspitze in den freien Blutstrom lässt sich eine Veränderung der optischen Intensität des registrierten Signals feststellen und der Messwert wird bevorzugt daraufhin überwacht, dass er im plausiblen Bereich liegt. Sobald das Auswertegerät diesen Zustand festgestellt hat, wird dem Anwender bevorzugt ein Signal für die korrekte Platzierung der Sonde gegeben.

In einem weiteren vorteilhaften Ausführungsbeispiel der vorliegenden Erfindung weist das längenverstellbare Schaftstück (30) einen Rohrabschnitt (35) auf, der in einem Innenraum (36) des Anschlussstücks (7) in Richtung des Faseroptiklumens verschiebbar ausgebildet ist.

Durch das Vorsehen eines Rohrabschnitts bzw. zylinderförmigen Abschnitts einerseits und einem dazu passenden Innenraum des Anschlussstücks oder Außenzylinder andererseits können diese beiden Elemente gegeneinander verschoben werden und bieten damit teleskopartig eine entsprechende Verlängerungsmöglichkeit für das Anschlussstück, an dessen proximalen Ende die faseroptische Sonde fest verbunden ist. Durch das Ineinanderschieben beispielsweise zweier aufeinander abgestimmter Zylinder kann das längenverstellbare Schaftstück und damit das Anschlussstück verlängert bzw. verkürzt werden. Auf diese Weise wird auch aus dem Anschlussstück bzw. dem längenverstellbaren Schaftstück herausragende Länge der faseroptischen Sonde verkürzt bzw. verlängert. Besonders bevorzugt werden durch das längenverstellbare Schaftstück verstellbare Bereiche von bis zu 50 mm, bevorzugt 20 bis 40 mm, besonders bevorzugt 30 mm maximale Verlängerung realisiert.

In einem weiteren vorteilhaften Ausführungsbeispiel der vorliegenden Erfindung weist der Rohrabschnitt (35) an seinem distalen Ende eine Dichtlippe (38) auf, über die der Rohrabschnitt (35) gegenüber dem Innenraum (36) des Anschlussstücks (7) abdichtbar ist.

Eine Dichtlippe kann zwischen den beiden gegeneinander zu bewegenden Teilen vorgesehen sein, um die Dichtigkeit zu erhöhen. Besonders bevorzugt werden hierfür Kunststoffe, Silicone, etc. eingesetzt, die den medizinischen Anforderungen an Toxizität und Sterilität genügen. Besonders bevorzugt ist die Dichtlippe an dem bevorzugt zylindrischen Rohrabschnitt an dem distalen Ende vorgesehen, so dass die Dichtlippe an der Innenfläche des Anschlussstücks bzw. des längenverstellbaren Schaftstücks zu liegen kommt und diesen Bereich abdichtet.

In einem weiteren vorteilhaften Ausführungsbeispiel der vorliegenden Erfindung weist das längenverstellbare Schaftstück (30) mindestens eine Längenmarkierung (26) auf.

Durch das Vorsehen einer Längenmarkierung auf dem längenverstellbaren Schaftstück ist es für den Operateur einfach möglich, die Länge einzustellen, die bei der vorliegenden faseroptischen Sonde und dem applizierten ZVK den korrekten Überstand des distalen Endes der faseroptischen Sonde über das distale Ende des Grundkörpers des ZVK sicherstellt. Diese Längenmarkierungen können besonderes bevorzugt farbig ausgebildet sein und einer farblichen Codierung auch des ZVK entsprechen.

In einem weiteren vorteilhaften Ausführungsbeispiel der vorliegenden Erfindung ist eine Arretiereinrichtung (27) zur Festlegung des längenverstellbaren Schaftstückes (30) vorgesehen.

Durch die Bereitstellung einer Arretiereinrichtung ist es möglich, das längenverstellbare Schaftstück nach Einstellen der gewünschten Längendifferenz in dieser Position bzw. dieser Längeneinstellung zu arretieren. Eine solche Arretiervorrichtung kann beispielsweise eine ausgebildete Kerbe und Nase sein, die in speziellen Positionen ineinander greifen und das längenverstellbare Schaftstück an dieser Stelle arretieren. Die Arretiereinrichtung kann besonders bevorzugt im nicht-sterilen Bereich angeordnet sein, d. h. auf der Außenfläche des Anschlussstücks bzw. des längenverstellbaren Schaftstücks. Besonders bevorzugt sind hier zusätzlich Schraubverschlüsse vorgesehen, die eine Arretierung durch Verschrauben sicherstellen. Besonders bevorzugt wird eine im unsterilen Bereich liegende Rasterung vorgesehen.

In einem weiteren vorteilhaften Ausführungsbeispiel der vorliegenden Erfindung weist die faseroptische Sonde (8) einen Ballon (28) an ihrem distalen Ende auf.

Bevorzugt weist mindestens eine faseroptische Sonde an ihrem distalen Ende einen kleinen, bei Bedarf mit Gas, vorzugsweise $CO_2$, oder mit einem Fluid, bevorzugt mit Kochsalzlösung, zu füllenden Ballon auf. Durch diesen Ballon kann die Spitze der Sonde mit dem Blutstrom in die rechte Herzkammer geschwemmt werden, wo sie verbleiben soll. Auf diese Weise ist es möglich, durch einen solchen Schwimmer bzw. Widerstand die distale Spitze der faseroptischen Sonde um 10 bis 15 cm in die rechte Herzkammer hineinzuschwemmen und dort Messungen der wirklich gemischtvenösen Sauerstoffsättigung in der rechten Herzkammer vorzunehmen. Der Ballon kann bevorzugt aufgeblasen bleiben, damit das distale Ende der Sonde mit Ballon durch den Blutstrom in der rechten Herzkammer verbleibt.

Bevorzugt kann alternativ die Ballon-Faseroptiksonde auch an der Spitze proximal hinter dem Ballon einen Thermistor zur Durchführung von Thermodilutionsmessungen aufweisen; die Injektion des thermalen Indikators erfolgt dabei bevorzugt über ein freies Lumen des Multilumen-ZVK in die obere Hohlvene. Daneben ist es auch möglich, dass die Ballon-Faseroptiksonde ein proximal hinter dem Ballon sich öffnendes Drucklumen aufweist, über das mittels Flüssigkeitssäule der Druck in der rechten Herzkammer kontinuierlich gemessen werden kann.

Alternativ kann der Druck in der rechten Herzkammer auch über den Ballon der Faseroptiksonde gemessen werden. Hierzu ist der Ballon besonders bevorzugt mit Flüssigkeit, vorzugsweise Kochsalzlösung, gefüllt. Hierbei addiert sich der zur Dehnung des Ballons erforderliche Druck zum von außen wirkenden intravasalen Druck in der rechten Herzkammer. Durch elektronische Verfolgung des Druckverlaufes beim Füllen des Ballons kann der Fülldruck nach Erreichen des gewünschten Ballonfüllungszustandes eliminiert und der reine intravasale Druck in der rechten Herzkammer ermittelt bzw. angezeigt werden. Vorteilhaft ist es, wenn der Ballon ein hohes Volumen/Füllungsdruck Verhältnis aufweist.

Bevorzugt kann in einem Ausführungsbeispiel der vorliegenden Erfindung auch ein Ballon der oben beschriebenen Art an einer anderen Sonde als der hier beschriebenen Faseroptiksonde befestigt sein, sei es eine andere Faseroptiksonde in einem anderen Kathetersystem oder eine andere funktionelle Sonde. Erfindungsgemäß kann die Ballonsonde auch mit anderen Katheterystemen als dem hier beschriebenen eingesetzt werden.

Besonders bevorzugt ist ein Kathetersystem vorgesehen, bei dem zwei Fiberoptiken enthalten sind, von der eine in einem Multilumenkatheter fest integriert zur Messung der Sauerstoffsättigung in der oberen Hohlvene vorgesehen ist und die Alternativsonde mit Ballon zur Messung der gemischtvenösen Sauerstoffsättigung.

In einem weiteren vorteilhaften Ausführungsbeispiel der vorliegenden Erfindung umfasst die faseroptische Sonde (8) mehr als eine optische Faser.

Gemäß einem weiteren Aspekt der Erfindung umfasst die faseroptische Sonde mehr als eine optische Faser, die durch dasselbe Faseroptiklumen applizierbar ist. Durch eine zweite optische Faser kann eine weitere Messung durchgeführt werden. Dies kann als Kontrollmessung in ungefähr demselben Bereich erfolgen oder aber an einem anderen Ort durch Vorsehen einer unterschiedlichen Länge der zweiten optischen Faser. Besonders bevorzugt weist das Kathetersystem ein weiteres Faseroptiklumen auf, durch das eine weitere faseroptische Sonde in den ZVK einbringbar ist. So ist es beispielsweise möglich, dass die erste faseroptische Sonde in der oberen Hohlvene (cava superior) liegt und dort die Sauerstoffsättigung in der oberen Hohlvene (Scs02) misst, während eine weitere Sonde in der rechten Herzkammer zu liegen kommt und die Sauerstoffsättigung in der rechten Herzkammer (Smv02) misst. Durch die Messung dieser beiden Parameter kann dann bevorzugt auch die Sauerstoffsättigung in der unteren Hohlvene (Sci02) abgeschätzt werden. Dies geschieht bevorzugt nach folgender Formel:

$$\mathtt{SmvO2 \;=\; (BFci \;x\; SciO2 \;+\; BFcs \;x\; ScsO2)/CO}$$

Wobei

BFci = Blufluss in der unteren Hohlvene (vena cava inferior)
BFcs = Blutfluss in der oberen Hohlvene (vena cava superior)
Sci02 = Sauerstoffsättigung in der unteren Hohlvene
Scs02 = Sauerstoffsättigung in der oberen Hohlvene
Smv02 = gemischtvenöse Sauerstoffsättigung in der rechten Herzkammer
CO = Herzzeitvolumen (cardiac output)= BFci + BFcs

Zur hinreichend genauen Abschätzung wird BFci angenommen mit 65% CO, BFcs mit 35% CO. Dies kann bevorzugt vom Benutzer eingegeben werden.
Demnach ergibt sich:

$$\mathtt{SmvO2 \;=\; 0,65\; SciO2 \;+\; 0,35\; ScsO2}$$

bzw.

$$\mathtt{SciO2 \;=\; (SmvO2 \;-\; 0,35\; ScsO2)/0,65}$$

Durch dieses Kathetersystem ist es möglich, dass zum Einsatz mit verschiedenen ZVKs nunmehr eine oder wenige Sonden mit unterschiedlicher Länge gefertigt und bereitgehalten werden müssen. Weiterhin ist durch die Platzierung von Alternativsonden mit dem Ballon in der rechten Herzkammer es möglich, wirklich gemischtvenöses Blut zu messen. Bei den herkömmlichen Kathetersystemen mit einer Sonde, bei der nur eine Spitze in der oberen Hohlvene liegt, wird die Sauerstoffsättigung auch nur in der oberen Hohlvene gemessen. Gerade bei vielen Schockzuständen wie bei Sepsis weichen aber die Sauerstoffsättigungen in der oberen und unteren Hohlvene ganz erheblich voneinander ab. In der unteren Hohlvene ist sie oft deutlich niedriger. Von wesentlich größerem klinischen Interesse ist deswegen die gemischtvenöse Sauerstoffsättigung im rechten Herzen, die durch das vorliegende Kathetersystem mit zwei faseroptischen Sonden gemessen werden können. Das Vorliegen unterschiedlicher Sauerstoffsättigungswerte Sci02 und Smv02 kann als Indiz für eine korrekte Positionierung der Sonden genutzt werden und bei unplausiblen Werten kann bevorzugt eine Warnung erfolgen.
Die Aufgabe wird auch gelöst durch ein Verfahren zur Applikation einer faseroptischen Sonde (8) in ein Kathetersystem (1) nach Anspruch 1 umfassend die Schritte:

Einbringen einer faseroptischen Sonde (8) in ein Anschlussstück (7),

Einstellen eines längenverstellbaren Schaftstückes (30) auf eine vorbestimmte Länge zur passgenauen Positionierung der faseroptischen Sonde (8) in dem Kathetersystem (1),

Fixieren der faseroptischen Sonde (8) in Bezug auf das Anschlussstück (7).

Durch das erfindungsgemäße Verfahren ist es möglich, einige wenige verschiedene Längen von Sonden einzusetzen und über das längenverstellbare Schaftstück die entsprechende Länge zum passenden ZVK voreinzustellen, bevor dann die faseroptische Sonde in das Kathetersystem eingebracht wird und in Bezug auf das Anschlussstück und damit innerhalb des Faseroptiklumens fixiert wird.

In einem weiteren vorteilhaften Ausführungsbeispiel der vorliegenden Erfindung ist zusätzlich der Schritt vorgesehen: Arretieren des längenverstellbaren Schaftstückes (30) auf einer vorbestimmten Länge.

Durch die Arretierung des längenverstellbaren Schaftstücks nach Einstellen der Länge, die der konkret applizierte ZVK erfordert, kann diese Länge nicht mehr während der weiteren Behandlung versehentlich verändert werden und damit ist die distale Spitze der faseroptischen Sonde gegenüber dem distalen Ende des Grundkörpers des ZVK festgelegt.

Bei Verwendung einer Sonde mit Ballon kann durch die Variation der Länge über das entsprechende Schaftstück das distale Ende mit Ballon dahin platziert werden, wo der Operateur dies für wünschenswert hält.

In einem weiteren Ausführungsbeispiel ist ein Verfahren vorgesehen, das weiter den Schritt umfasst:

Messung einer Intensität der reflektierten optischen Strahlung in der faseroptischen Sonde (8) während des Einbringens der Faseroptik mit dem angeschlossenen Auswertegerät für die Messung der Sauerstoffsättigung; Überprüfung der Plausibilität der gemessenen Intensitäts-Werte.

Bevorzugt kann damit während des Einführens der Faseroptik mit dem angeschlossenen Auswertegerät für die Messung der Sauerstoffsättigung die Intensität der reflektierten optischen Strahlung gemessen und die Plausibilität des erhaltenen Sauerstoffsättigungswertes überprüft werden. Bei Eintritt der Sondenspitze in den freien Blutstrom lässt sich eine Veränderung der optischen Intensität des registrierten Signals feststellen und der Messwert wird bevorzugt daraufhin überwacht, dass er im plausiblen Bereich liegt. Sobald das Auswertegerät diesen Zustand festgestellt hat, wird dem Anwender bevorzugt ein Signal für die korrekte Platzierung der Sonde gegeben.

In einem weiteren Ausführungsbeispiel ist ein Verfahren vorgesehen, das weiter den Schritt umfasst: Ausgabe eines Signals, wenn die faseroptische Sonde (8) korrekt platziert ist.

Als Signal können Anzeigen auf einem Monitor, akustische Signale, optische Signale, etc. genutzt werden. Die korrekte Platzierung ergibt sich bevorzugt aus der Plausibilität der gemessenen Werte. Wenn beim Vorschieben der Sonde ein solcher plausibler Bereich erreicht ist, wird ein Signal ausgegeben, das die korrekte Platzierung der faseroptischen Sonde anzeigt.

In den Figuren wird ein Ausführungsbeispiel der vorliegenden Erfindung beschrieben. Hierbei zeigen:

Fig. 1a    eine mehrfach unterbrochene Teilansicht eines zentralvenösen Multilumenkatheters, welcher Teil eines erfindungsgemäßen Kathetersystems ist;

Fig. 1b    eine Ansicht einer erfindungsgemäßen faseroptischen Sonde;

Fig. 2    eine mittels gestrichelten Kreis K in Fig. 1b angedeutete Teilansicht im Querschnitt, welche im Wesentlichen das Anschlussstück mit längenverstellbarem Schaftstück zeigt und

Fig. 3    eine schematische Ansicht des distalen Endes des Grundkörpers eines Multilumenkatheters mit den daran herausragenden Faseroptiksonden gemäß einem Ausführungsbeispiel der vorliegenden Erfindung.

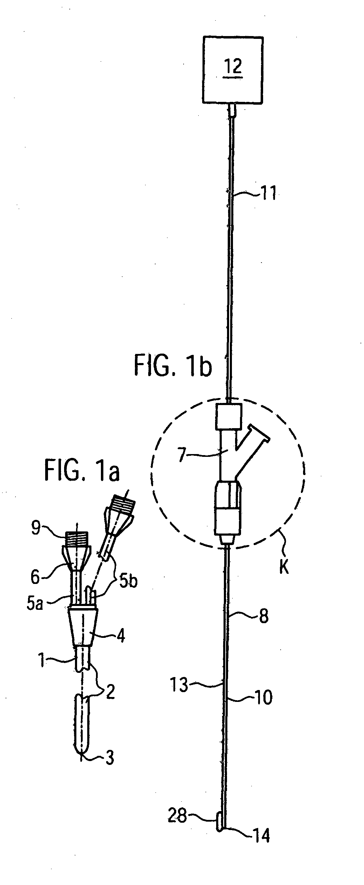

In Figur 1a ist eine zentralvenöser Multilumenkatheter 1 dargestellt, der einen flexiblen, länglichen zentralvenös applizierbaren, unterbrochen dargestellten Grundkörper 2 aufweist, in welchem mehrere Lumina ausgebildet sind, deren distale Öffnungen (nicht dargestellt) am distalen Ende 3 des Grundkörpers 2 bzw. in der Nähe des distalen Endes 3 des Grundkörpers 2 angeordnet sind. Proximal verlaufen die Lumina oberhalb einer Verzweigung 4 in mehreren Fortsätzen 5A, 5B weiter, wobei Fortsatz 5B unterbrochen dargestellt ist. Das Faseroptiklumen (nicht sichtbar), das im Durchmesser deutlich größer ist als der Außendurchmesser des distalen Teils 10 der Faseroptiksonde 8, verläuft vom distalen Ende 3 des Kathetergrundkörpers 2 durch diesen hindurch und weiter durch den Fortsatz 5A zu einem Gegenstück 6 für das Anschlussstück 7 der Faseroptiksonde 8. Über den Fortsatz 5A und der Verzeigung 4 ist das Gegenstück 6 fest mit dem Grundkörper 2 verbunden. Das Gegenstück 6 weist ein Außengewinde 9 auf, über welches das Anschlussstück 7 nach Einführen des distalen Teils der Faseroptiksonde in das Faseroptiklumen formschlüssig angeschlossen werden kann. Über den Fortsatz 5A genauso wie über den Fortsatz 5B sind Faseroptiksonden in das Kathetersystem einführbar.

In **Figur 1b** ist eine Faseroptiksonde 8 dargestellt, die sich zur Messung der zentralvenösen Sauerstoffsättigung eignet. Hierzu ist sie über proximal in einem Kabel 11 verlaufende optische Faser an eine Lichtquellen- und Messvorrichtung 12 angeschlossen, welche zur gleichzeitigen Aussendung und Messung von Strahlung ausgebildet ist und bevorzugt über eine Auswerteeinheit verfügt. Distal vom Anschlussstück 7 aus gesehen, verlaufen die optischen Fasern in einem dünnen, flexibeln Schaft 13, welcher in der Nähe seines abgerundeten distalen Endes 14 mit einem antithrombogenen Überzug versehen ist. Die Länge des distalen Teils 10 ist auf die Länge des Faseroptiklumens des Multilumenkatheters 1 durch Justage des längenverstellbaren Schaftstücks des Anschlussstücks 7 abgestimmt. Mit der faseroptischen Sonde 8 ist das Anschlussstück 7 fest verklebt. Am distalen Ende 14 der Faseroptiksonde 8 ist ein Ballon 28 vorgesehen, der bedarfsweise mit Gas gefüllt werden kann.

Die faseroptische Sonde 8 aus Figur 1b wird im Gebrauch dann über beispielsweise den Fortsatz 5A in den Katheter und damit in den Grundkörper 2 eingeführt. Über das Gegenstück 6 und das Außengewinde 9 wird dieser Fortsatz fest mit dem Anschlussstück 7 durch ein Luer-Lock verbunden.

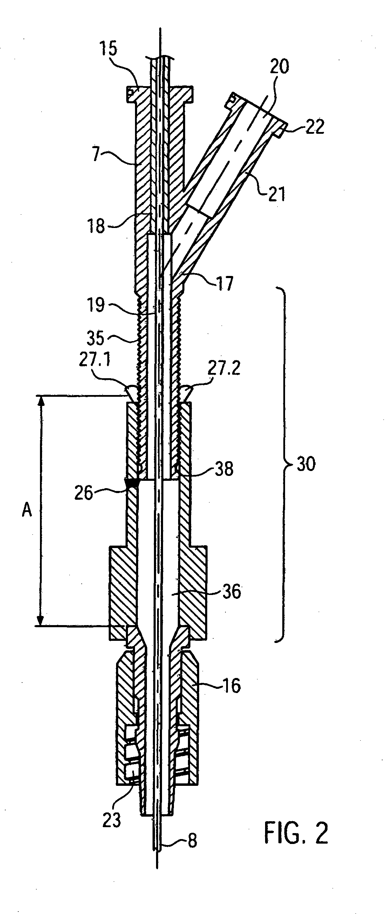

In **Figur 2** ist im Querschnitt das Anschlussstück 7 mit dem längenverstellbaren Schaftstück 30 dargestellt. Das Anschlussstück 7 besteht aus drei miteinander verklebten Teilen 17, 18 und 19. Die faseroptische Sonde (8) ist mit dem Abschlussteil 15 fest verklebt. Über eine ausgebildete Hülse 35 ist eine verstellbare Verbindung zu dem Teil 16 hergestellt. Die Hülse 35 kann in dem vorgesehenen zylindrischen Innenraum 36 entlang des eingezeichneten Doppelpfeils A hin und her bewegt werden. Da die faseroptische Sonde 8 fest mit dem Teil 18 bzw. dem Abschlussteil 15 verklebt ist, wird die faseroptische Sonde durch das Verschieben der Hülse 35 in dem zylindrischen Innenraum 36 innerhalb des Faseroptiklumens nach vorne und hinten verschoben. Damit wird das distale Ende des Faseroptiklumens durch ein Verschieben entlang des Doppelpfeils A in Bezug auf das distale Ende des Grundkörpers 2 des Katheters 1 in seinem Überstand über dieses Ende variiert. Auf diese Weise ist es möglich, das distale Ende der Faseroptiksonde 8 in Bezug auf die Länge des Grundkörpers 2 des Katheters 1 genau einzustellen. Auf dem Grundkörper 16 ist eine Längenmarkierung 26 vorgesehen. Die Hülse 35 ist in der Abbildung bis zu dieser Längenmarkierung 26 vorgeschoben worden. Über die Arretiereinrichtungen 27.1 und 27.2 wird die Hülse 35 in dem zylindrischen Innenraum 36 genau an dieser Stelle, die mit der Längenmarkierung 26 definiert ist, festgehalten. Auf diese Weise kann der vorgewählte Abstand arretiert werden und das längenverstellbare Schaftstück 30 des Anschlussstücks 7 auf eine für den verwendeten zentralvenösen Katheter 1 abgestimmte Länge der faseroptischen Sonde 8 eingestellt werden. Um die Dichtigkeit zu erhöhen, ist eine Dichtlippe 38 vorgesehen.

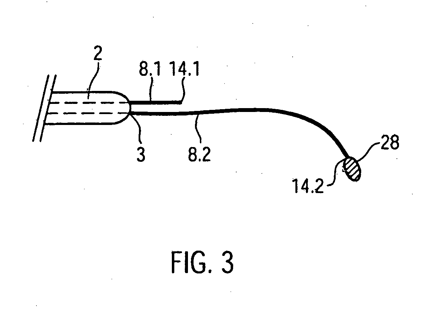

In **Figur 3** ist schematisch das distale Ende des Grundkörpers 2 eines Katheters gezeigt. Am distalen Ende 3 des Grundkörpers 2 ragen zwei faseroptische Sonden 8.1 und 8.2 heraus. Das distale Ende der ersten faseroptischen Sonde 14.1 liegt zirka 25 mm hinter dem distalen Ende 3 des Grundkörpers 2. Das distale Ende 14.2 der faseroptischen Sonde 8.2 liegt zirka 15 cm hinter dem distalen Ende 3 des Grundkörpers 2. Am distalen Ende 14.2 der faseroptischen Sonde 8.2 ist ein Ballon 28 dargestellt, der aufgeblasen ist. Auf diese Weise wird durch den Blutstrom die faseroptische Sonde 8.2 in dieser Stellung stabilisiert und kann in die rechte Herzkammer geschwemmt und dort stabilisiert werden. Dort kann dann wirklich gemischtvenöses Blut gemessen werden.

Bezugszeichenliste

| | |
|---|---|
| 1 | Multilumenkatheter/ Kathetersystem |
| 2 | Grundkörper |
| 3 | Distales Ende des Grundkörpers 2 |
| 4 | Verzweigung |
| 5 | Fortsatz |
| 6 | Gegenstück für Anschlussstück 7 |
| 7 | Anschlussstück |
| 8 | Faseroptiksonde |
| 9 | Außengewinde |
| 10 | Distales Teil der Faseroptiksonde 10 |
| 11 | Kabel |
| 12 | Lichtquellen- und Messvorrichtung |
| 13 | Flexibler Schaft |
| 14 | Distales Ende der Faseroptiksonde 8 |
| 15 | Abschlussteil |
| 16 | Gewindeteil |
| 17 | Y-Teil des Anschlussstück 7 |
| 18 | verklebtes Teil |
| 19 | Inneres des Y-Teils 17 |
| 20 | Spülkanal |
| 21 | Spülstutzen |
| 22 | Flansch |

| 23 | Innengewinde des Gewindeteils 16 |
|----|----|
| 25 | Befestigungsmittel |
| 26 | Längenmarkierung |
| 27 | Arretiereinrichtung |
| 28 | Ballon |
| 30 | Längenverstellbares Schaftstück des Anschlussstücks 7 |
| 35 | Hub/ Kolben/ Stempel |
| 36 | Innenraum des Zylinders |
| 38 | Dichtlippe |

**Patentansprüche**

1. Katheter system aufweisend
   einen flexiblen, länglichen, zentralvenös applizierbaren Grundkörper (2)
   eine faseroptische Sonde (8)
   ein Faseroptiklumen zur Aufnahme der faseroptischen Sonde (8) und Befestigungsmittel (25) zur Vermeidung einer Längsverschiebung der faseroptischen Sonde (8) relativ zu dem Faseroptiklumen, wobei die Befestigungsmittel (25) lösbar sind, um eine Längsverschiebung der faseroptischen Sonde (8) relativ zu dem Faseroptiklumen zur Entnahme der optischen Sonde (8) zuzulassen, wobei
   die Befestigungsmittel (25) ein fest mit der faseroptischen Sonde (8) verbundenes Anschlussstück (7) und ein fest mit dem zentralvenös applizierbaren Grundkörper (2) verbundenes Gegenstück (6) aufweisen, welche aneinander anschließbar sind und wobei in angeschlossenem Zustand der Abstand zwischen distalem Ende (3) des zentralvenös applizierbaren Grundkörpers (2) und distalem Ende (14) der faseroptischen Sonde (8) durch die festen Verbindungen zwischen der faseroptischen Sonde (8) und dem Anschlussstück (7) sowie Grundkörper (2) und Gegenstück (6) auf einen vorbestimmten Wert eingestellt ist
   **dadurch gekennzeichnet, dass**
   zusätzlich ein längenverstellbares Schaftstück (30) am Anschlussstück (7) vorgesehen ist, über das die Länge des Anschlussstückes (7) und damit die Länge des Faseroptiklumens variiert werden kann.

2. Katheter system nach Anspruch 1, wobei das längenverstellbare Schaftstück (30) einen Rohrabschnitt (35) aufweist, der in einem Innenraum (36) des Anschlussstücks (7) in Richtung des Faseroptiklumens verschiebbar ausgebildet ist.

3. Katheter system nach Anspruch 2, wobei der Rohrabschnitt (35) an seinem distalen Ende eine Dichtlippe (38) aufweist, über die der Rohrabschnitt (35) gegenüber dem Innenraum (36) des Anschlussstücks (7) abdichtbar ist.

4. Katheter system nach einem der vorhergehenden Ansprüche, wobei das längenverstellbare Schaftstück (30) mindestens eine Längenmarkierung (26) aufweist.

5. Katheter system nach einem der vorhergehenden Ansprüche, wobei eine Arretiereinrichtung (27) zur Festlegung des längenverstellbaren Schaftstückes (30) vorgesehen ist.

6. Katheter system nach einem der vorhergehenden Ansprüche, wobei die faseroptische Sonde (8) einen Ballon (28) an ihrem distalen Ende aufweist.

7. Katheter system nach einem der vorhergehenden Ansprüche, wobei die faseroptische Sonde (8) mehr als eine optische Faser umfasst.

8. Verfahren zur Applikation einer faseroptischen Sonde (8) in ein Katheter system (1) nach Anspruch 1 umfassend die Schritte:

   Einbringen einer faseroptischen Sonde (8) in ein Anschlussstück (7),
   Einstellen eines längenverstellbaren Schaftstückes (30) auf eine vorbestimmte Länge zur passgenauen Positionierung der faseroptischen Sonde (8) in dem Katheter system (1),
   Fixieren der faseroptischen Sonde (8) in Bezug auf das Anschlussstück (7).

9. Verfahren nach Anspruch 8 weiter umfassend den Schritt:

Arretieren des längenverstellbaren Schaftstückes (30) auf einer vorbestimmten Länge.

10. Verfahren nach einem der Ansprüche 8 oder 9 weiter umfassend die Schritte:

Messung einer Intensität der reflektierten optischen Strahlung in der faseroptischen Sonde (8) während des Einbringens der Faseroptik mit dem angeschlossenen Auswertegerät für die Messung der Sauerstoffsättigung; Überprüfung der Plausibilität der gemessenen Intensitäts-Werte.

11. Verfahren nach Anspruch 10 weiter umfassend den Schritt:

Ausgabe eines Signal, wenn die faseroptische Sonde (8) korrekt platziert ist.

**Claims**

1. A catheter system comprising:

a flexible, elongated base body (2) adapted to be applied to a vein central-venously;
a fibre-optic probe (8);
a fibre-optic lumen for receiving the fibre-optic probe (8); and
attachment means (25) configured to avoid a longitudinal displacement of the fibre-optic probe (8) relative to the fibre-optic lumen and configured to detach so as to allow removal of the optic probe (8) through the longitudinal displacement of the fibre-optic probe (8) relative to the fibre-optic lumen, the attachment means (25) having a connector piece (7) firmly connected to the fibre-optic probe (8) and a counter-piece (6) firmly connected to the base body (2) adapted to be applied to a vein central-venously,
wherein the connector piece (7) is connectable to the counter-piece (6),
wherein in the connected state, the distance between the distal end (3) of the base body (2) adapted to be applied to a vein central-venously and the distal end (14) of the fibre-optic probe (8) is adjusted to a predetermined value by the firm connections between the fibre-optic probe (8) and the connector piece (7) and between the base body (2) and the counter-piece (6);
**characterized in that** additionally an adjustable lengthwise shaft piece (30) is provided on the connector piece (7), wherein the shaft piece (30) is arranged to vary the length of the connector piece (7) and thereby the length of the fibre-optic lumen.

2. Catheter system according to claim 1, wherein the adjustable lengthwise shaft piece (30) includes a tube section (35) displaceable in a direction of the fibre-optic lumen in an inner space (36) of the connector piece (7).

3. Catheter system according to claim 2, wherein at its distal end, the tube section (35) includes a sealing lip (38), wherein at the inner space (36) the tube section (35) is sealed off via the sealing lip (38).

4. Catheter system according to any of the preceding claims, wherein the adjustable lengthwise shaft piece (30) includes at least one length mark (26).

5. Catheter system according to any of the preceding claims, wherein there is provided a locking device (27) configured to fix the adjustable lengthwise shaft piece (30).

6. Catheter system according to any of the preceding claims, wherein at its distal end, the fibre-optic probe (8) includes a balloon (28).

7. Catheter system according to any of the preceding claims, wherein the fibre-optic probe (8) includes more than one optical fibre.

8. A method for an application of a fibre-optic probe (8) in a catheter system (1) according to claim 1, comprising the steps:

introducing a fibre-optic probe (8) into a connector piece (7);
setting an adjustable lengthwise shaft piece (30) to a predetermined length so as to accurately position the fibre-optic probe (8) in the catheter system (1);
fixing the fibre-optic probe (8) with reference to the connector piece (7).

**9.** The method according to claim 8, further comprising the step:

locking the adjustable lengthwise shaft piece (30) at a predetermined length.

**10.** The method according to claim 8 or 9, further comprising the steps:

measuring an intensity of the reflected optical radiation in the fibre-optic probe (8) during the introduction of the fibre optic with the connected evaluation apparatus for the measurement of oxygen saturation; checking the plausibility of measured intensity values.

**11.** The method according to claim 10, further comprising the step:

emitting a signal if the fibre-optic probe (8) is correctly placed.

**Revendications**

**1.** Système de cathéter présentant
un corps de base (2) flexible, longitudinal, applicable au niveau veineux central,
une sonde à fibres optiques (8),
un lumen à fibres optiques pour recevoir la sonde à fibres optiques (8) et des moyens de fixation (25) pour empêcher un décalage longitudinal de la sonde à fibres optiques (8) par rapport au lumen à fibres optiques, dans lequel les moyens de fixation (25) sont amovibles pour permettre un décalage longitudinal de la sonde à fibres optiques (8) par rapport au lumen à fibres optiques pour retirer la sonde optique (8), dans lequel
les agents de fixation (25) présentent un raccord (7) relié fixement à la sonde à fibres optiques (8) et un élément opposé (6) relié fixement au corps de base (2) applicable au niveau veineux central, qui peuvent être raccordés l'un à l'autre et dans lequel, à l'état raccordé, l'intervalle entre l'extrémité distale (3) du corps de base applicable au niveau veineux central (2) et l'extrémité distale (14) de la sonde à fibres optiques (8) est ajusté à une valeur prédéterminée, par les liaisons fixes entre la sonde à fibres optiques (8) et le raccord (7) et entre le corps de base (2) et l'élément opposé (6),
**caractérisé en ce que**
en outre, une tige (30) réglable au niveau longitudinal est prévue sur le raccord (7) par lequel on peut faire varier la longueur du raccord (7) et, ainsi, la longueur du lumen à fibres optiques.

**2.** Système de cathéter selon la revendication 1, dans lequel la tige réglable au niveau longitudinal (30) présente un segment tubulaire (35) qui est conçu de façon à pouvoir être décalé dans un espace intérieur (36) du raccord (7) en direction du lumen à fibres optiques.

**3.** Système de cathéter selon la revendication 2, dans lequel le segment tubulaire (35) présente au niveau de son extrémité distale une lèvre d'étanchéité (38) par laquelle le segment tubulaire (35) peut être étanchéifié par rapport à l'espace intérieur (36) du raccord (7).

**4.** Système de cathéter selon l'une des revendications précédentes, dans lequel la tige réglable longitudinalement (30) présente au moins une marque longitudinale (26).

**5.** Système de cathéter selon l'une des revendications précédentes, dans lequel un dispositif d'arrêt (27) pour fixer la tige réglable longitudinalement (30) est prévu.

**6.** Système de cathéter selon l'une des revendications précédentes, dans lequel la sonde à fibres optiques (8) présente un ballonnet (28) à son extrémité distale.

**7.** Système de cathéter selon l'une des revendications précédentes, dans lequel la sonde à fibres optiques (8) comprend plus d'une fibre optique.

**8.** Procédé pour l'application d'une sonde à fibres optiques (8) dans un système de cathéter (1) selon la revendication 1, comprenant les étapes suivantes :

l'insertion d'une sonde à fibres optiques (8) dans un raccord (7),

le réglage d'une tige réglable longitudinalement (30) à une longueur prédéterminée pour placer exactement la sonde à fibres optiques (8) dans le système de cathéter (1),
la fixation de la sonde à fibres optiques (8) par rapport au raccord (7).

9. Procédé selon la revendication 8, comprenant en outre l'étape suivante :

l'arrêt de la tige réglable longitudinalement (30) à une longueur prédéterminée.

10. Procédé selon l'une des revendications 8 ou 9, comprenant en outre les étapes suivantes :

la mesure d'une intensité du rayonnement optique réfléchi dans la sonde à fibres optiques (8) pendant l'insertion de la fibre optique avec l'appareil d'analyse raccordé pour la mesure de la saturation en oxygène ;
le contrôle de la plausibilité des valeurs d'intensité mesurées.

11. Procédé selon la revendication 10, comprenant en outre l'étape suivante :

l'émission d'un signal lorsque la sonde à fibres optiques (8) est placée correctement.

FIG. 1b

FIG. 1a

FIG. 2

FIG. 3

**IN DER BESCHREIBUNG AUFGEFÜHRTE DOKUMENTE**

**In der Beschreibung aufgeführte Patentdokumente**

- EP 1402917 A
- WO 2007101508 A1